# EUROPEAN PATENT SPECIFICATION

(11) **EP 0 579 802 B1**
(45) Date of publication and mention of the grant of the patent: **26.04.2000**
(21) Application number: 93902658.9
(22) Date of filing: 10.12.1992
(51) Int. Cl.: A61K 49/00, C07F 11/00, C07F 15/00, C07D 471/10, C07K 2/00, C07D 471/08, C07D 487/08, A61K 47/48

(54) **BICYCLE-POLYAZAMACROCYCLOCARBOXYLIC ACID COMPLEXES, CONJUGATES, PREPARATION AND USE AS CONTRAST AGENTS**
BICYLCLISCHE, POLYAZAMAKROCYCLOCARBONSÄURE-KOMPLEXE, KONJUGATE, HERSTELLUNG UND VERWENDUNG ALS KONTRASTSTOFFE
COMPLEXES ET CONJUGUES A BASE D'ACIDES BICYCLOPOLYAZAMACROCYCLOCARBOXYLIQUE, LEUR PREPARATION ET UTILISATION ET TANT QU'AGENTS DE CONTRASTE

(30) Priority: 10.12.1991 US 805270
(43) Date of publication of application: 26.01.1994
(73) Proprietor: THE DOW CHEMICAL COMPANY, Midland, Michigan 48674 (US)
(72) Inventor: KIEFER, Garry, E., Lake Jackson, TX 77566 (US); SIMON, Jaime, Angleton, TX 77515 (US)
(74) Representative: Raynor, John
(86) International application number: US9211003
(87) International publication number: WO9311800

(56) References cited:
- EP-A- 0 298 939
- EP-A- 0 352 218
- EP-A- 0 391 766
- EP-A- 0 438 206
- WO-A-91/10645
- US-A- 4 920 195
- US-A- 4 933 441
- US-A- 4 963 344
- JOURNAL OF HETEROCYCLIC CHEMISTRY vol. 27, no. 2 , February 1990 , TAMPA pages 167 - 169 HARRI TAKALO ET. AL.
- STETTER et al., Tetrahedron, Vol. 37, pp. 767-772, 1981, "Darstellung und Komplexbildung von Polyazacycloalkan-N-Essigsaeuren".

## Description

This invention concerns complexes that contain as the ligand bicyclopolyazamacrocyclocarboxylic acids, and conjugates thereof, for use as contrast agents in magnetic resonance imaging (MRI). Processes for preparing these complexes and conjugates are also provided. To better understand this invention, a brief background on MRI is provided in the following section.

### Background

MRI is a non-invasive diagnostic technique which produces well resolved cross-sectional images of soft tissue within an animal body, preferably a human body. This technique is based upon the property of certain atomic nuclei (e.g. water protons) which possess a magnetic moment [as defined by mathematical equations; see G M. Barrow, Physical Chemistry, 3rd Ed., McGraw-Hill, NY (1973)] to align in an applied magnetic field. Once aligned, this equilibrium state can be perturbed by applying an external radio frequency (RF) pulse which causes the protons to be tilted out of alignment with the magnetic field. When the RF pulse is terminated, the nuclei return to their equilibrium state and the time required for this to occur is known as the relaxation time. The relaxation time consists of two parameters known as spin-lattice (T1) and spin-spin (T2) relaxation and it is these relaxation measurements which give information on the degree of molecular organization and interaction of protons with the surrounding environment.

Since water content of living tissue is substantial and variations in content and environment exist among tissue types, diagnostic images of biological organisms are obtained which reflect proton density and relaxation times. The greater the differences in relaxation times (T1 and T2) of protons present in tissue being examined, the greater will be the contrast in the obtained image [*J. Magnetic Resonance* 33, 83-106 (1979)].

It is known that paramagnetic chelates possessing a symmetric electronic ground state can dramatically affect the T1 and T2 relaxation rates of juxtaposed water protons and that the effectiveness of the chelate in this regard is related in part, to the number of unpaired electrons producing the magnetic moment [*Magnetic Resonance Annual* 231-266 (Raver Press NY (1985)]. It has also been shown that when a paramagnetic chelate of this type is administered to a living animal, its effect on the T1 and T2 of various tissues can be directly observed in the magnetic resonance (MR) images with increased contrast being observed in the areas of chelate localization. It has therefore been proposed that stable, non-toxic paramagnetic chelates be administered to animals in order to increase the diagnostic information obtained by MRI [*Frontiers of Biol. Energetics* I, 752-759 (1978); *J. Nucl. Med.* 25, 506-513(1984); Proc. of NMR Imaging Symp. (Oct. 26-27, 1980); F. A. Cotton et al., *Adv*. *Inorg*. *Chem*. 634-639 (1966)]. Paramagnetic metal chelates used in this manner are referred to as contrast enhancement agents or contrast agents.

There are a number of paramagnetic metal ions which can be considered when undertaking the design of an MRI contrast agent. In practice, however, the most useful paramagnetic metal ions are gadolinium (Gd⁺³) iron (Fe⁺³), manganese (Mn⁺²) and (Mn⁺³), and chromium (Cr⁺³), because these ions exert the greatest effect on water protons by virtue of their large magnetic moments. In a non-complexed form (e.g. GdCl₃), these metal ions are toxic to an animal, thereby precluding their use in the simple salt form. Therefore, a fundamental role of the organic chelating agent (also referred to as a ligand) is to render the paramagnetic metal non-toxic to the animal while preserving its desirable influence on T1 and T2 relaxation rates of the surrounding water protons.

Art in the MRI field is quite extensive, such that the following summary, not intended to be exhaustive, is provided only as a review of this area and other compounds that are possibly similar in structure. U.S. Patent 4,899,755 discloses a method of alternating the proton NMR relaxation times in the liver or bile duct of an animal using Fe⁺³-ethylene-bis(2-hydroxyphenylglycine) complexes and its derivatives, and suggests among various other compounds the possible use of a pyridine macrocyclomethylenecarboxylic acid. U.S. Patent 4,880,008 (a CIP of U.S. Patent 4,899,755) discloses additional imaging data for liver tissue of rats, but without any additional complexes being shown. U.S. Patent 4,980,148 disclose gadolinium complexes for MRI which are non-cyclic compounds. C. J. Broan et al., *J. Chem Soc*., *Chem. Commun*., 1739-1741 (1990) describe some bifunctional macrocyclic phosphinic acid compounds. C. J. Broan et al., *J. Chem. Soc., Chem. Commun*., 1738-1739(1990) describe compounds that are triazabicyclo compounds. I. K. Adzamli et al., *J. Med. Chem*. 32, 139-144 (1989) describes acyclic phosphonate derivatives of gadolinium complexes for NMR imaging

At the present time, the only commercial contrast agent available in the U.S. is the complex of gadolinium with diethylenetriaminepentaacetic acid (DTPA-Gd³⁺-MAGNEVIST™ by Shering). MAGNEVIST™ is considered as a non-specific/perfusion agent since it freely distributes in extracellular fluid followed by efficient elimination through the renal system MAGNEVIST™ has proven to be extremely valuable in the diagnosis of brain lesions since the accompanying breakdown of the blood/brain barrier allows perfusion of the contrast agent into the affected regions. In addition to MAGNEVIST™, Guerbet is commercially marketing a macrocyclic perfusion agent (DOTAREM™) which presently is only available in Europe. A number of other potential contrast agents are in various stages of development.

It would be advantageous if contrast agents were developed that could have site specificity for the tissue desired to be imaged, rather than non-specific/perfusion agents. The present invention is directed to just such novel complexes comprising a ligand that is a bicyclo-polyazamacrocyclocarboxylic acid of the formula 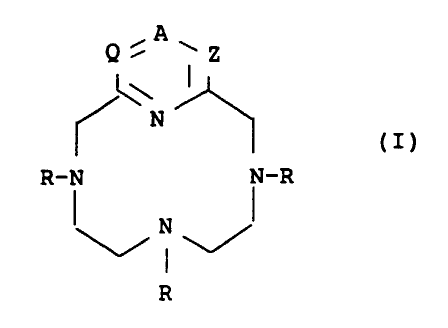 wherein:
R is 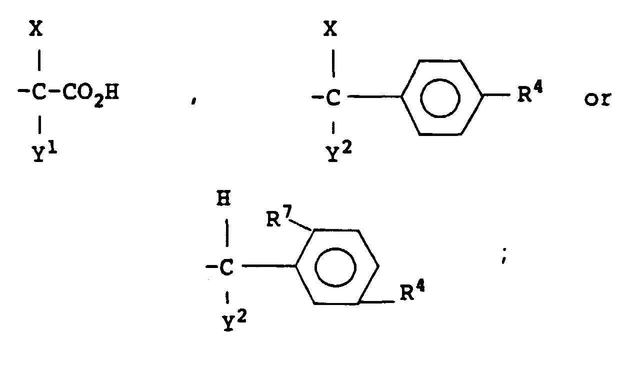 where:
X and Y¹ are independently H, OH or C₁-C₃ alkyl;
Y² is H or COOH;
R⁷ is H, OH or OCH₃; and
R⁴ is H, NO₂, NH₂, isothiocyanato, semicarbazido, thiosemicarbazido, maleimido, bromoacetamido or carboxyl; with the proviso that at least two R terms must be 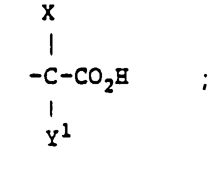
A = CH, N, C-Br, C-Cl, C-OR¹, C-OR², N⁺-R³ X⁻, or 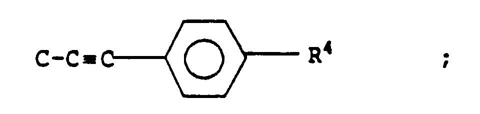
R¹ = H, C₁-C₅ alkyl, benzyl, or benzyl substituted with at least one R⁴;
R² is C₁-C₁₆ alkylamino;
R³ is C₁-C₁₆ alkyl, benzyl, or benzyl substituted with at least one R⁴;
R⁴ is defined as before;
X⁻ is Cl⁻; Br⁻, I⁻ or H₃CCO₂⁻;
Q and Z independently are CH, N, N⁺-R³ X⁺, C-CH₂-OR¹ or C-C(O)-R⁵;
R¹ and R³ are defined as above;
R⁵ is-O-(C₁-C₃ alkyl), OH or NHR⁶;
R⁶ is C₁-C₅ alkyl or a biologically active material;
X⁻ is defined as above; and
   with the provisos that:
   a) when Q, A or Z is N or N⁺-R³X⁻; then the other two groups must be CH;
   b) when A is C-Br, C-Cl, C-OR¹ or C-OR², then both Q and Z must be CH;
   c) the sum of the R², R⁴ and R⁶ terms, when present, may not exceed one, and one of the terms R², R⁴ and R⁶ must be present; and
   d) only one of Q or Z can be C-C(O)-R⁵ and when one of Q or Z is C-C(O)-R⁵, then A must be CH; and
      complexed with a metal ion selected from Gd⁺³, Mn⁺² or Fe⁺³; or
      pharmaceutically-acceptable salts thereof.

Bifunctional complexes of Formula (I) are desirable to prepare the conjugates of this invention. Such ligands must have at least one of R², R⁴ or R⁶ present:
one R term is 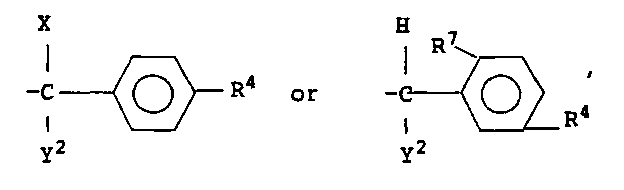
where X, Y², R⁴ and R⁷ are defined as above; or
A is C-OR¹, C-OR², where R¹ and R² are defined as above or 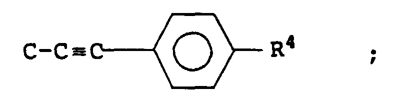
where R⁴ is defined as above; or
A is CH, and one of Q or Z is CH and the other is C-C(O)-R⁵ or C-CH₂-OR¹, where R¹ and R⁵ are defined as above; especially perferred are those ligands where R⁵ is NHR⁶, where R⁶ is a biologically active material.

The ligands of Formula (I) are complexed with various metal ions, such as gadolinium (Gd⁺³), iron (Fe⁺³), and manganese (Mn⁺²), and Gd⁺³ being preferred The complexes so formed can be used by themselves or can be attached, by being covalently bonded, to a larger molecule, such as a dextran, a polypeptide or a biologically active molecule, including an antibody or fragment thereof, and used for diagnostic purposes. Such conjugates and complexes are useful as contrast agents.

The complexes and conjugates of this invention can be modified to provide a specific overall charge. For example, when the metal ion is + 3 the following can be obtained:
(A) an overall neutral charge - when
   R is 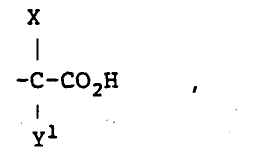
   and X and Y¹ are all equal to H;
(B) an overall + 1 charge - when
   one of A, Q or Z is N⁺-R³ X⁻, where R³ and X⁻ are defined as above; and the three R terms are 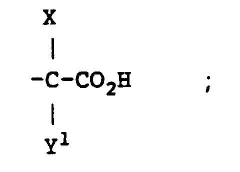
   and X and Y¹ are all equal to H.

Both the complexes and conjugates may be formulated to be in a pharmaceutically acceptable form for administration to an animal.

Use of the ligands of this invention with other metal ions for diagnosis of disease states such as cancer is possible. The use of those complexes and conjugates is discussed in another copending application.

The complex has the ligand of Formula (I) numbered for nomenclature purposes as follows:

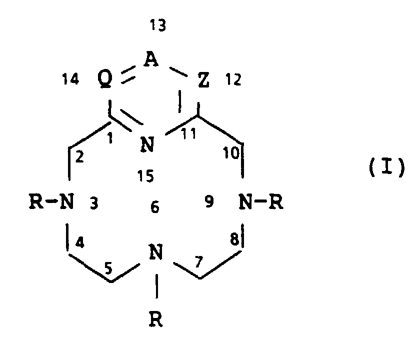

The present invention concerns development of contrast agents having a neutral or + 1 charge which enables site specific delivery of the contrast agent to a desired tissue. The advantage being increased contrast in the areas of interest based upon tissue affinity as opposed to contrast arising from non-specific perfusion which may or may not be apparent with an extracellular agent. The specificity of the ligand of Formula (I) may be controlled by adjusting the total charge and lipophilic character of the complex. The overall range of the charge of the complex is from + 1 to 0. For example, for a complex having a + 1 overall charge has heart uptake expected; whereas when the overall charge of the complex is 0 (thus neutral), the complex may have the ability to cross the blood brain barrier and normal brain uptake may be possible.

Tissue specificity may also be realized by ionic or covalent attachment of the chelate to a naturally occurring or synthetic macromolecule having specificity for a desired target tissue. One possible application of this approach is through the use of chelate conjugated monoclonal antibodies which would transport the paramagnetic chelate to diseased tissue enabling visualization by MRI. In addition, attachment of a paramagnetic chelate to a macromolecule can further increase the contrast agent efficiency resulting in improved contrast relative to the unbound chelate. Recent work by Lauffer (U.S. Patents 4,880,008 and 4,899,755) has demonstrated that variations in lipophilicity can result in tissue-specific agents and that increased lipophilic character favors non-covalent interactions with blood proteins resulting in enhancement of relaxivity.

Additionally, the present contrast agents of Formula (I) which are neutral in charge are particularly preferred for forming the conjugates of this invention since undesirable ionic interactions between the chelate and protein are minimized which preserves the antibody immunoreactivity. Also the present neutral complexes reduce the osmolarity relative to DTPA-Gd⁺³, which may alleviate the discomfort of injection.

While not wishing to be bound by theory, it is believed that when a charged complex of the invention is made (e.g. + 1 for heart), the variations in that chelate ionic charge can influence biolocalization. Thus, if the antibody or other directing moiety is also specific for the same site, then the conjugate displays two portions to aid in site specific delivery.

The terms used in Formula (I) and for this invention are further defined as follows. "C₁-C₃ alkyl", "C₁-C₅ alkyl", "C₁-C₁₈ alkyl", include both straight and branched chain alkyl groups. An "animal" includes a warmblooded mammal, preferably a human being.

"Biologically active material" refers to, for example, a dextran, peptide, or molecules that have specific affinity for a receptor, or preferably antibodies or antibody fragments.

"Antibody" refers to any polyclonal, monoclonal, chimeric antibody or heteroantibody, preferably a monoclonal antibody; "antibody fragment" includes Fab fragments and F(ab')₂ fragments, and any portion of an antibody having specificity toward a desired epitope or epitopes. When using the term "radioactive metal chelate/antibody conjugate" or "conjugate", the "antibody" is meant to include whole antibodies and/or antibody fragments, including semisynthetic or genetically engineered variants thereof. Possible antibodies are 1116-NS-19-9 (anti-colorectal carcinoma), 1116-NS-3d (anti-CEA), 703D4 (anti-human lung cancer), 704A1 (anti-human lung cancer), CC49 (anti-TAG-72), CC83 (anti-TAG-72) and B72.3. The hybridoma cell lines 1116-NS-19-9, 1116-NS-3d, 703D4, 704A1, CC49, CC83 and B72.3 are deposited with the American Type Culture Collection, having the accession numbers ATCC HB 8059, ATCC CRL 8019, ATCC HB 8301, ATCC HB 8302, ATCC HB 9459, ATCC HB 9453 and ATCC HB 8108, respectively.

As used herein, "complex" refers to a complex of the compound of the invention, e.g. Formula (I), complexed with a metal ion, where at least one metal atom is chelated or sequestered; "conjugate" refers to a metal ion chelate that is covalently attached to an antibody or antibody fragment. The terms "bifunctional coordinator", "bifunctional chelating agent" and "functionalized chelant" are used interchangeably and refer to compounds that have a chelant moiety capable of chelating a metal ion and a moiety covalently bonded to the chelant moiety that is capable of serving as a means to covalently attach to an antibody or antibody fragment.

The bifunctional chelating agents described herein (represented by Formula I) can be used to chelate or sequester the metal ions so as to form metal ion chelates (also referred to herein as "complexes"). The complexes, because of the presence of the functionalizing moiety (represented by R², R⁴ or R⁶ in Formula I), can be covalently attached to biologically active materials, such as dextran, molecules that have specific affinity for a receptor, or preferably covalently attached to antibodies or antibody fragments. Thus the complexes described herein may be covalently attached to an antibody or antibody fragment or have specific affinity for a receptor and are referred to herein as "conjugates".

As used herein, "pharmaceutically-acceptable salt" means any salt or mixtures of salts of a complex or conjugate of formula (I) which is sufficiently non-toxic to be useful in therapy or diagnosis of animals, preferably mammals. Thus, the salts are useful in accordance with this invention. Representative of those salts formed by standard reactions from both organic and inorganic sources include, for example, sulfuric, hydrochloric, phosphoric, acetic, succinic, citric, lactic, maleic, fumaric, palmitic, cholic, palmoic, mucic, glutamic, gluconic, d-camphoric, glutaric, glycolic, phthalic, tartaric, formic, lauric, steric, salicylic, methanesulfonic, benzenesulfonic, sorbic, picric, benzoic, cinnamic acids and other suitable acids. Also included are salts formed by standard reactions from both organic and inorganic sources such as ammonium or 1-deoxy-1-(methylamino)-D-glucitol, alkali metal ions, alkaline earth metal ions, and other similar ions. Particularly preferred are the salts of the complexes or conjugates of formula (I) where the salt is potassium, sodium or ammonium. Also included are mixtures of the above salts.

The complexes or conjugates of the present invention contain a ligand of Formula (I). The ligands are prepared by various processes. Typical general synthetic approaches to such processes are provided by the reaction schemes given below.

In Scheme 1, the compounds of Formula (I) are prepared wherein Q, A and Z = CH, and all three R =

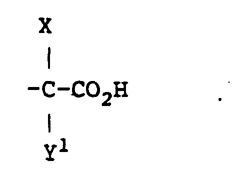

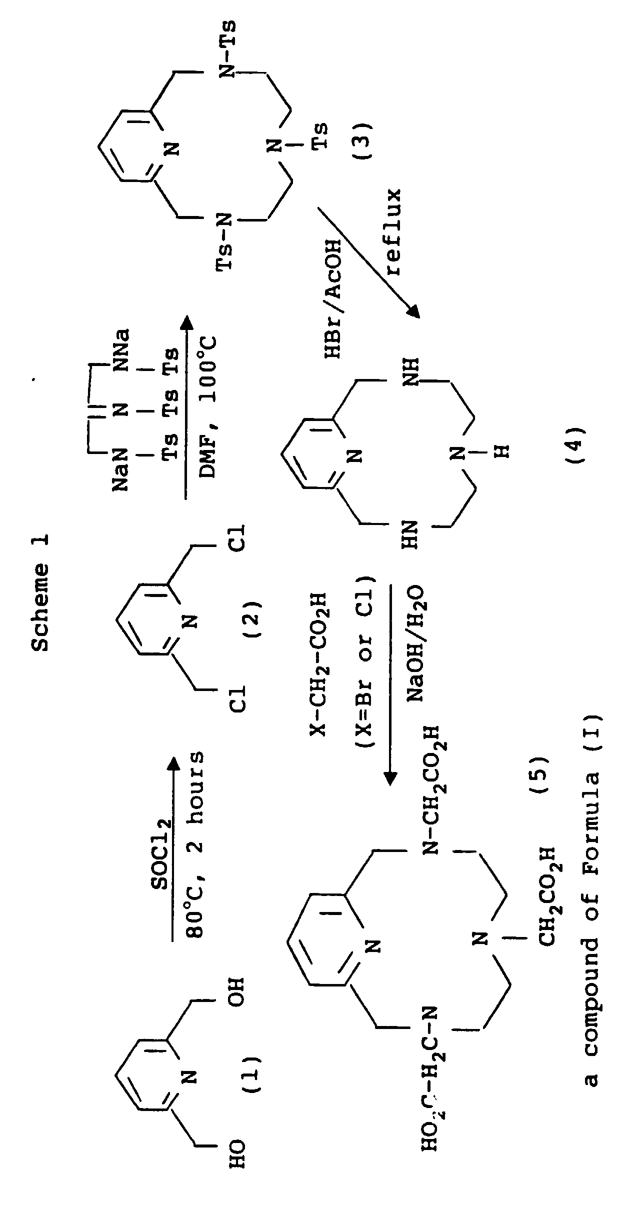

Scheme 2 prepares the compounds of Formula (I) wherein A = C-Br, and Q and Z = CH.

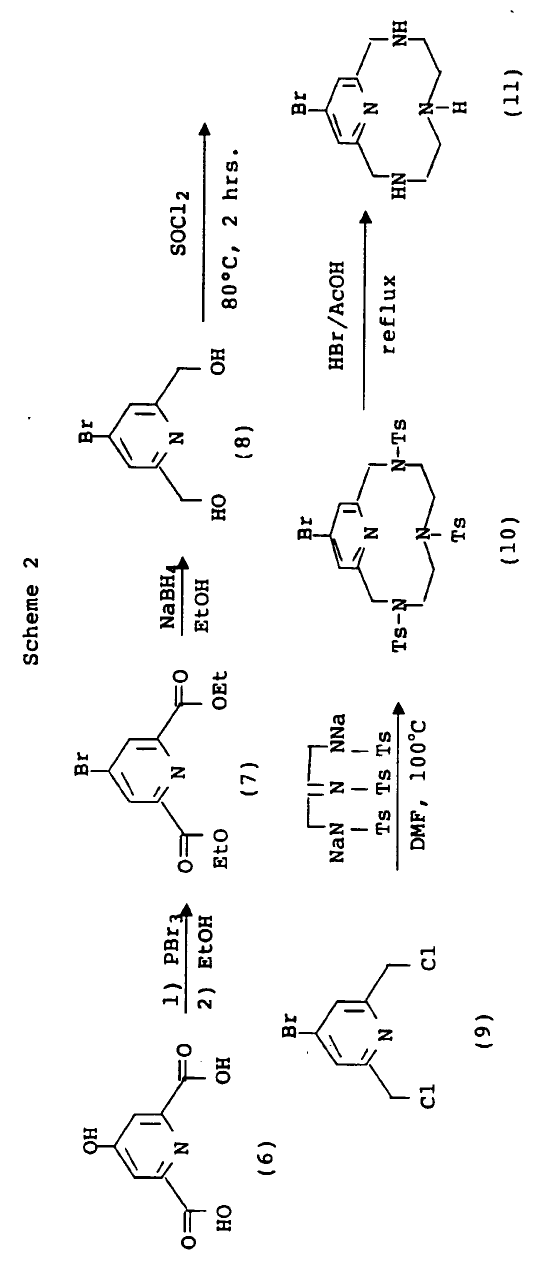

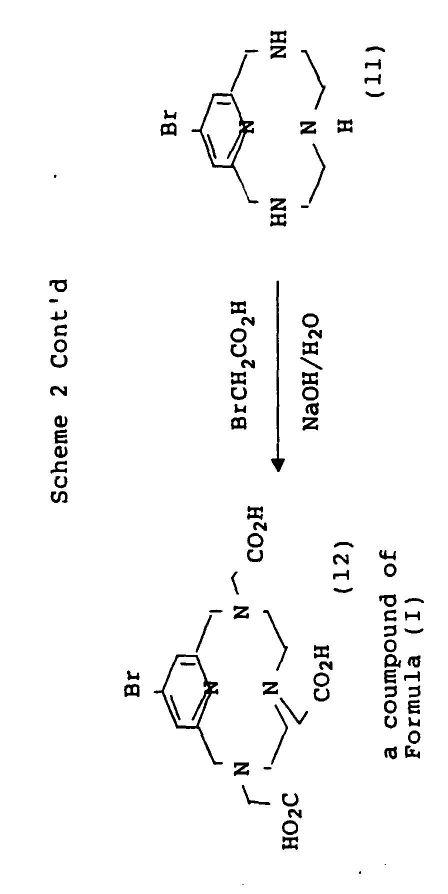

Scheme 3 prepares the compounds of Formula (I) wherein A = 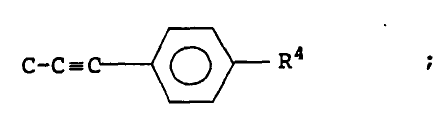 R⁴ = H, NO₂, NH₂ or SCN; and Q and Z = CH.

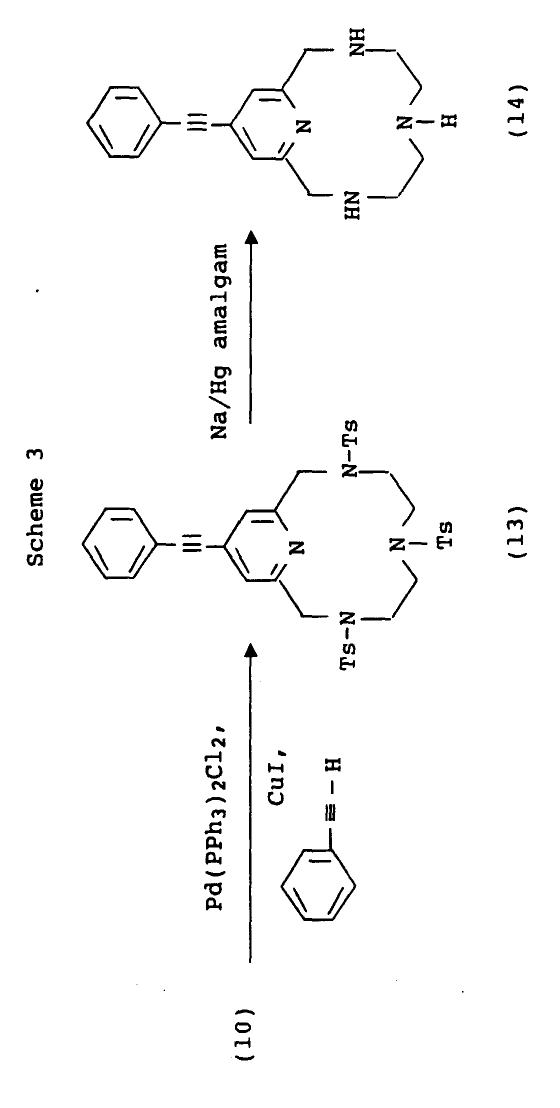

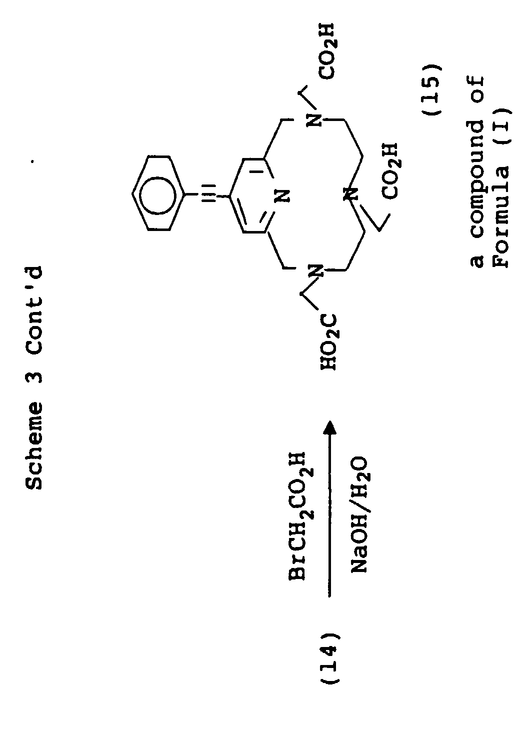

Scheme 4 prepares the compounds of Formula (I) wherein A = C-OR², where R² = C₁-C₅ alkylamino; and
Q and Z = CH.

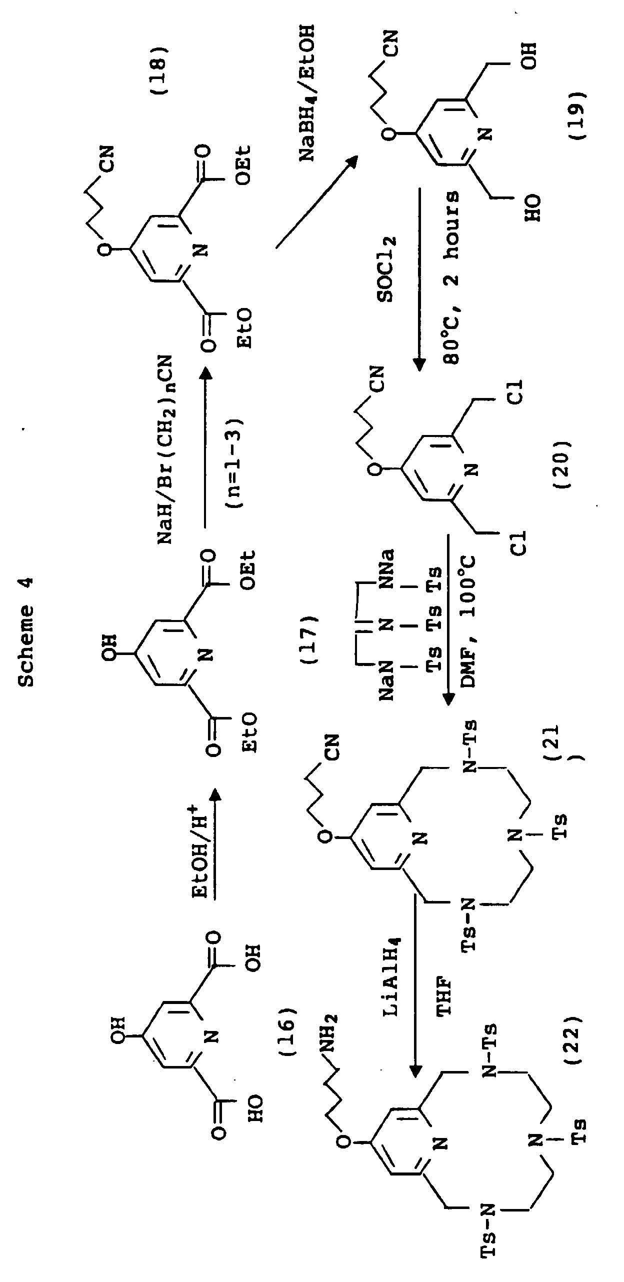

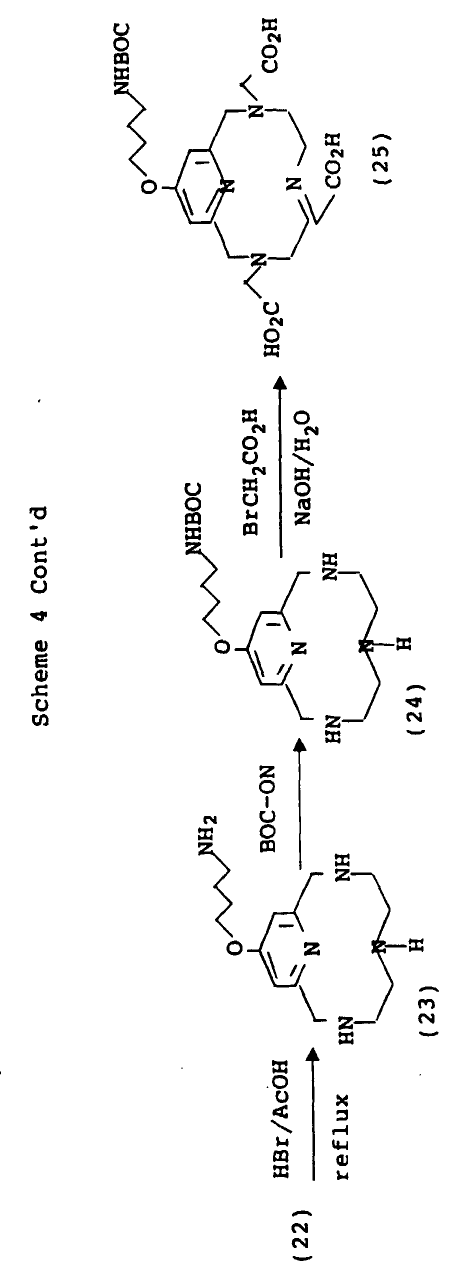

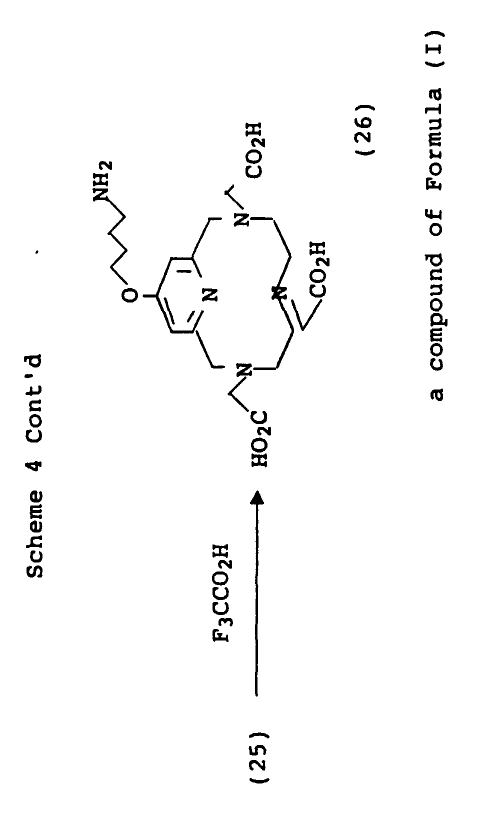

Scheme 5 prepares the compounds of Formula (I) wherein A = CH; and one of Q or Z = CH and the other Q or Z = C-C(O)-R⁶ or C-CH₂-R⁶, where R⁶ is defined as before.

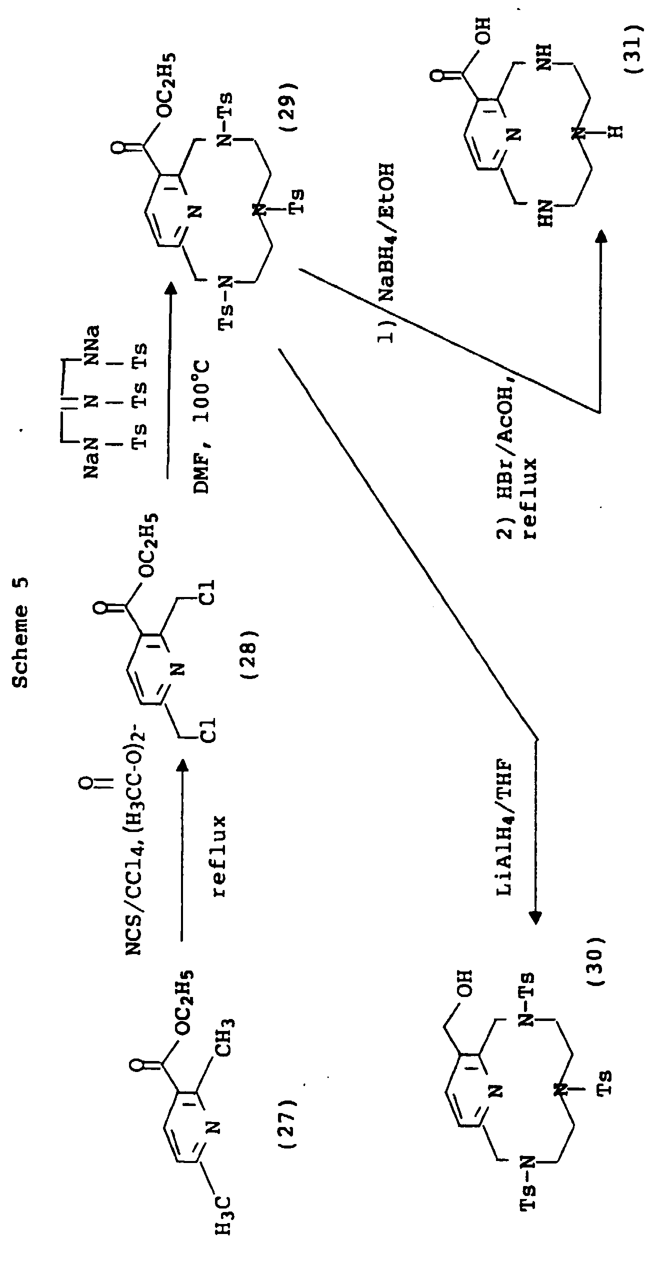

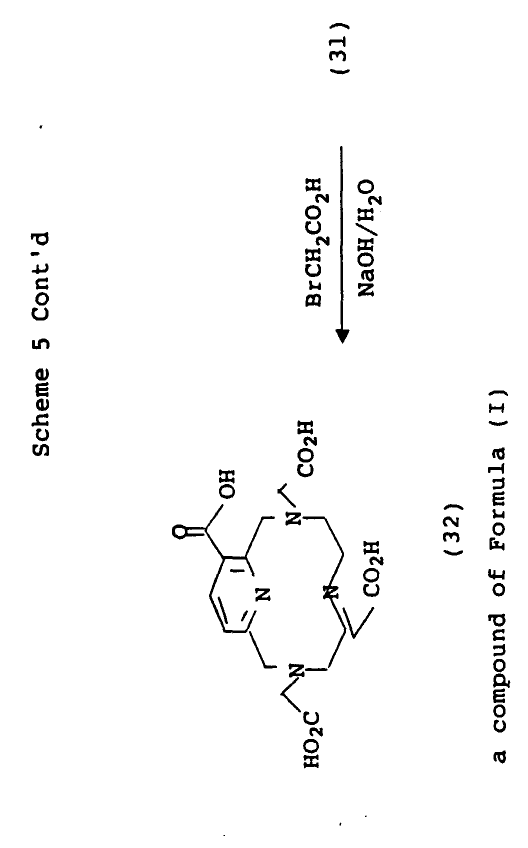

Scheme 6 prepares the compounds of Formula (I) wherein Z = C-CH₂-OBz or C-C(O)-R⁵ where R⁵ = -O-(C₁-C₃ alkyl), OH or NHR⁶, where is defined as before; and Q and A = CH.

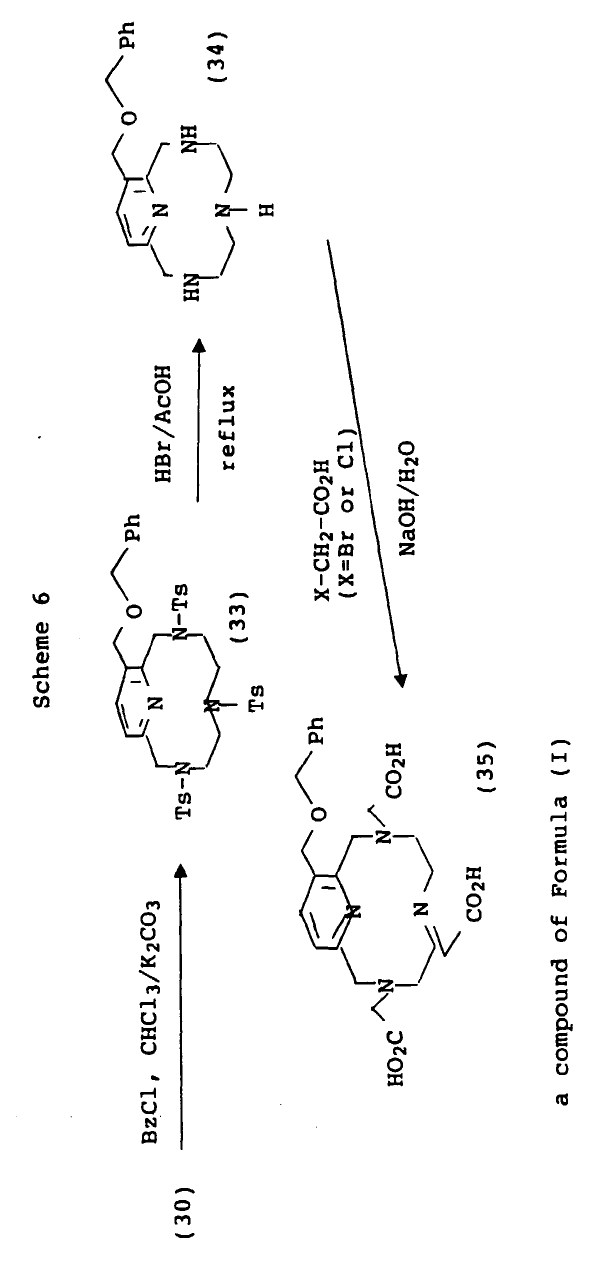

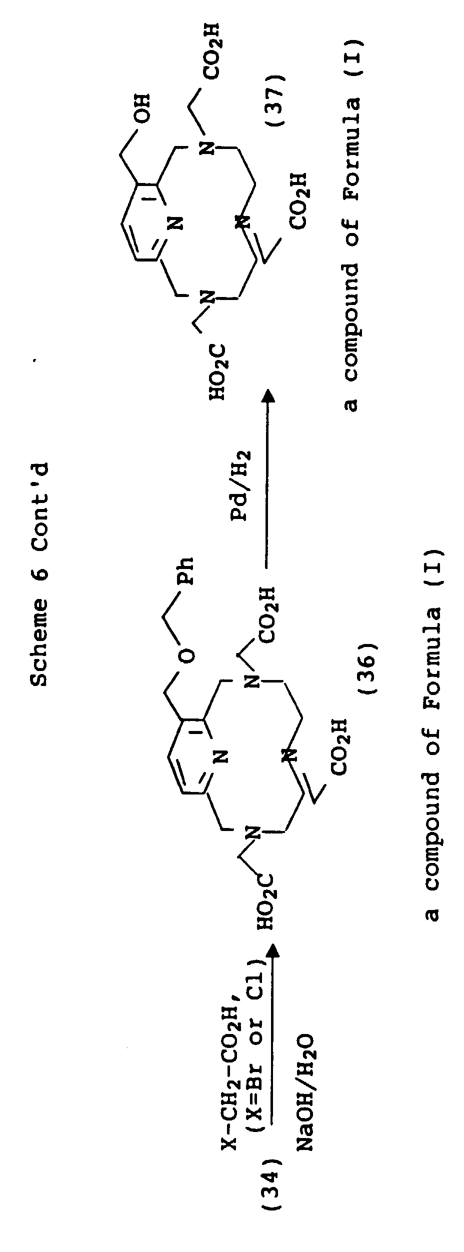

Scheme 7 prepares the compounds of Formula (I) wherein A = N or N⁺ -R⁵ X⁻; R⁵ = C₁-C₁₆ alkyl and is X⁻ halide; and
Q and Z = CH.

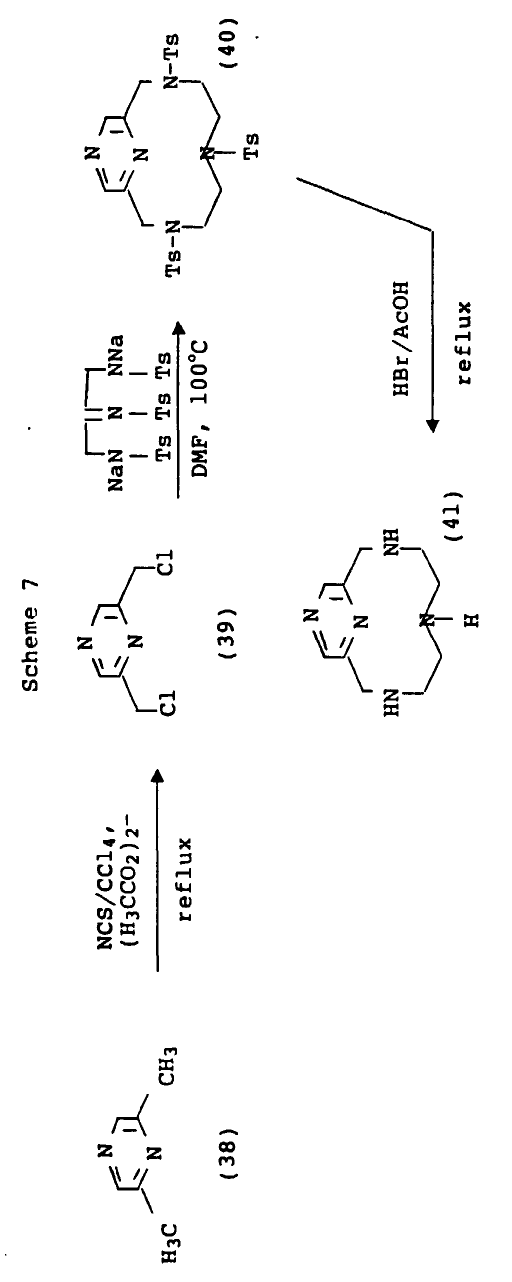

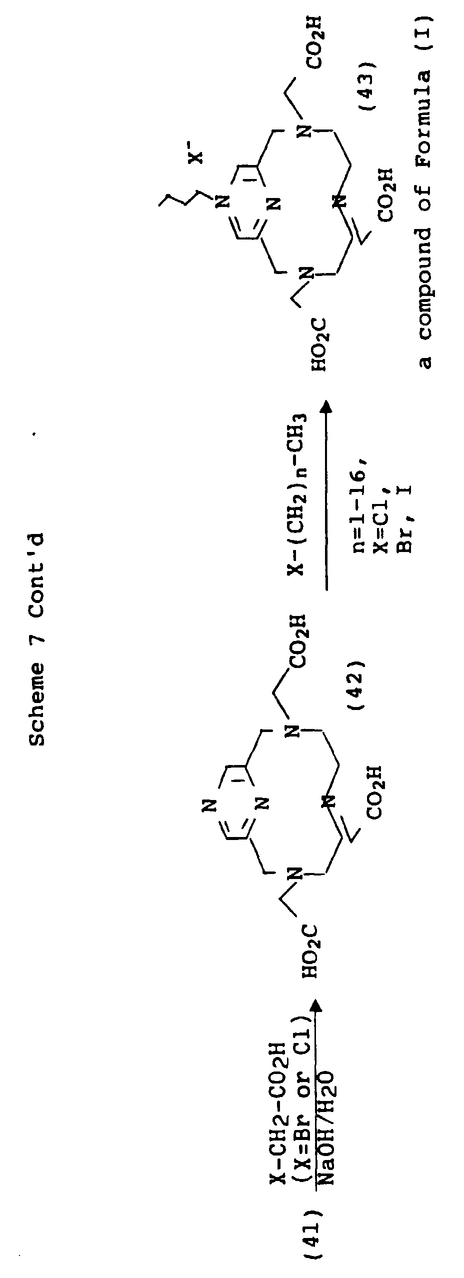

Scheme 8 prepares the compounds of Formula (I) wherein Q = N⁺-R⁵X⁻; where R⁵ = C₁-C₁₆ alkyl and X⁻ = halide; and
A and Z = CH.

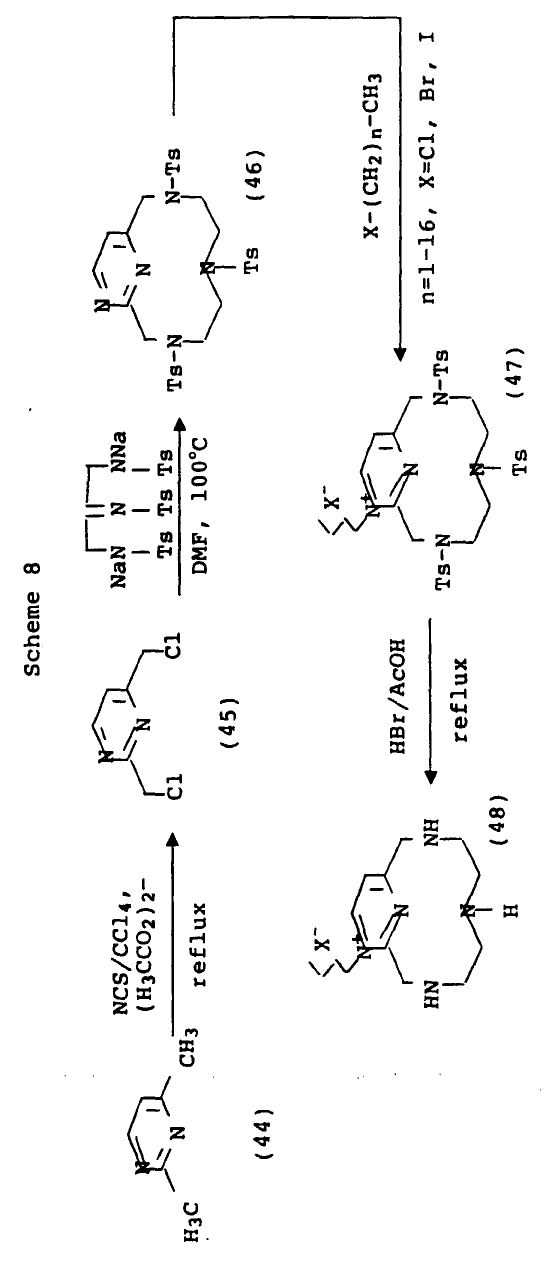

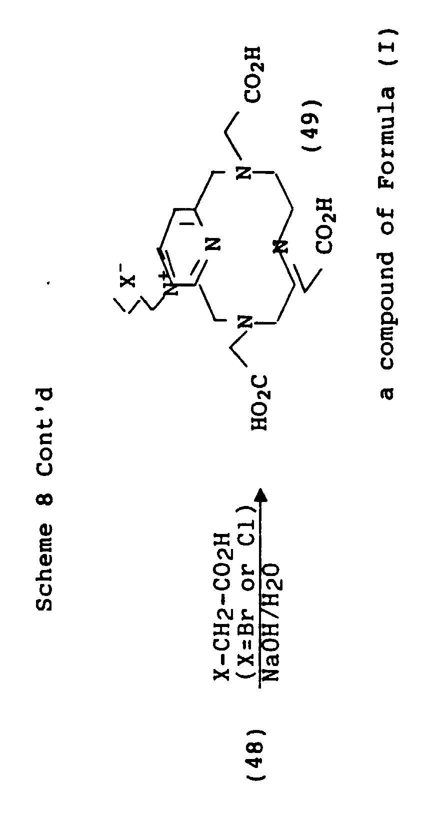

Scheme 9 prepares the compounds of Formula (I) wherein
Q = N or N+-R⁵ X⁻, where R⁵ = C₁-C₁₆ alkyl and
X⁻ = halide; and
A and Z = CH.

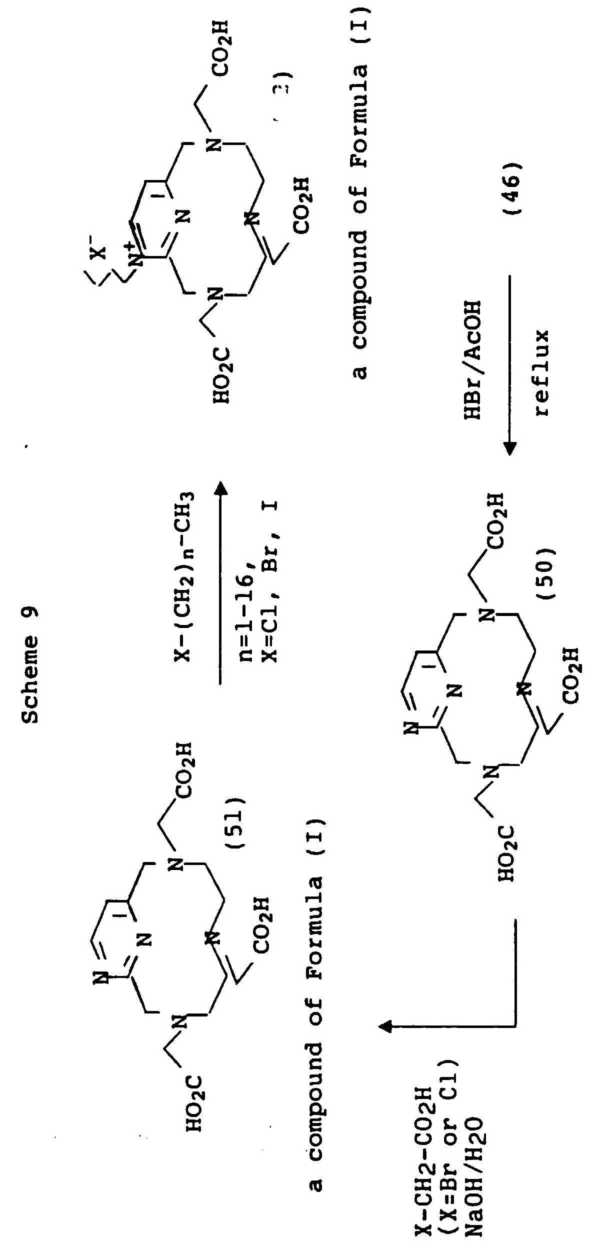

Scheme 10 prepares the compounds of Formula (I) wherein R term at the 6 position is 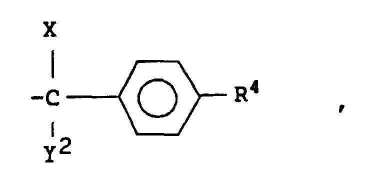 where R⁴ = NO₂ or NH₂;
Y² = CO₂H (or with a change of reagent Y² = H); and
A, Q and Z = CH.

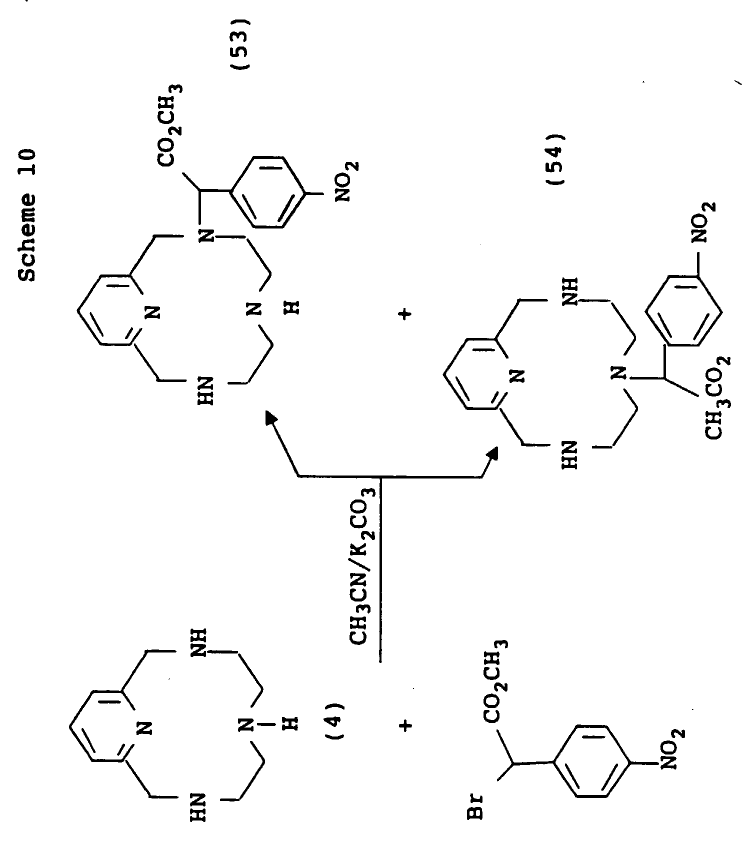

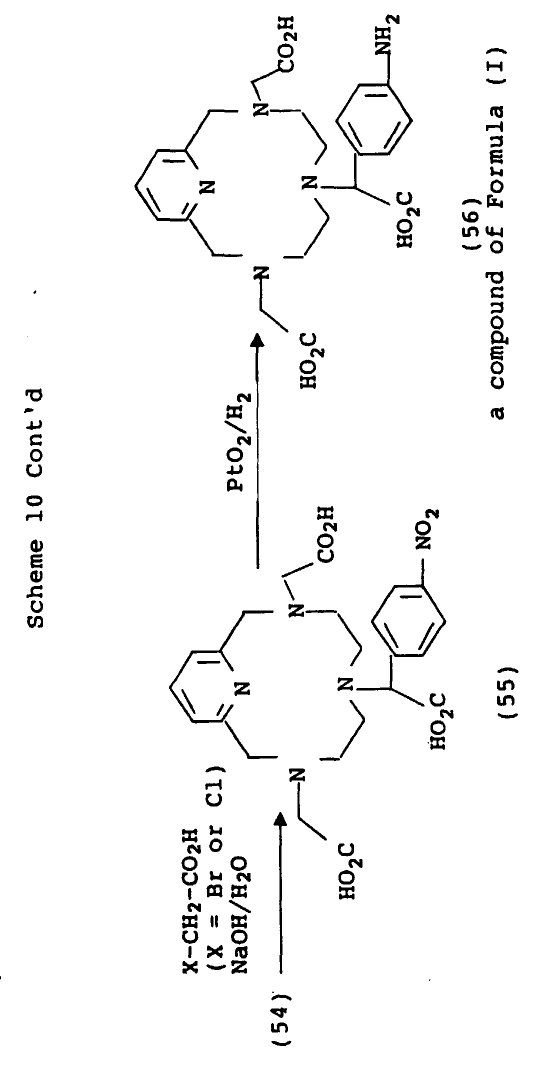

Scheme 11 prepares the compounds of Formula (I) wherein the R term at the 9 position is 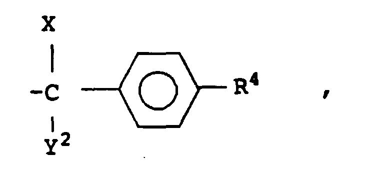 where R⁴ = NO₂ or NH₂;
Y² = CO₂H (or with a change of reagent Y² = H); and
A, Q and Z = CH.

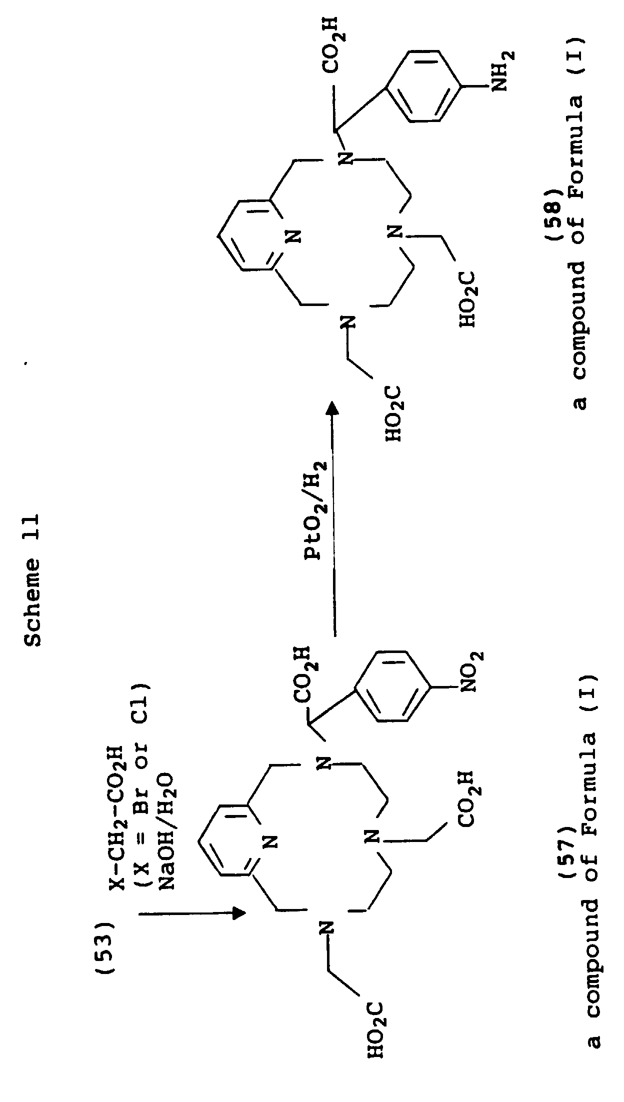

Scheme 12 prepares the compounds of Formula (I) wherein n = 1 (but would also apply if n = 2 or 3 with the corresponding change in the reagent), the R term at the 6 position has T = 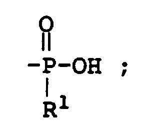 where R¹ = -OH; and X and Y = H;
the R term at the 3 and 9 positions have T = COOH;
R⁷ = OH or OCH₃; and
A,Q and Z = CH.

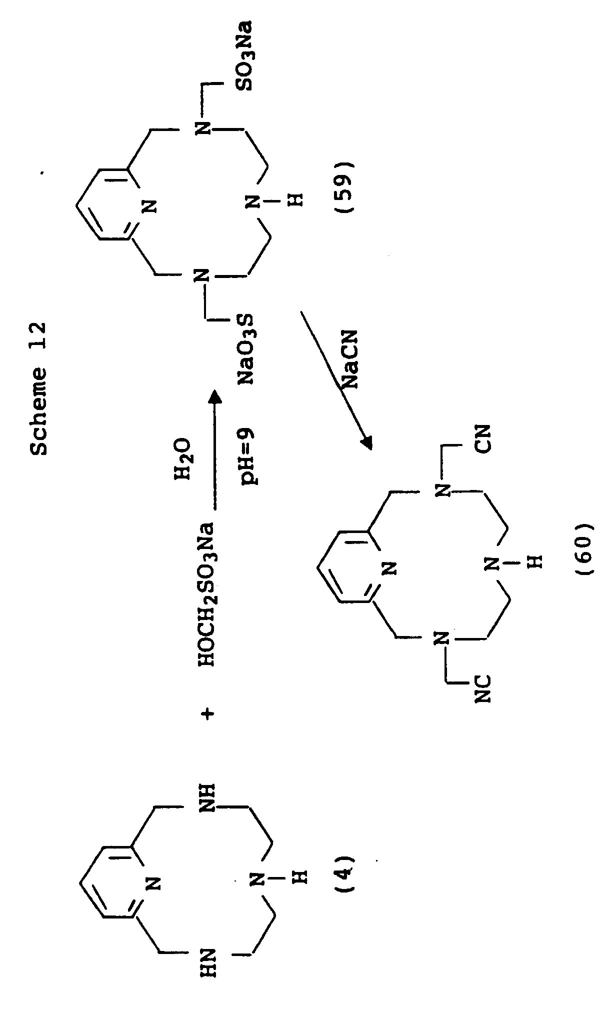

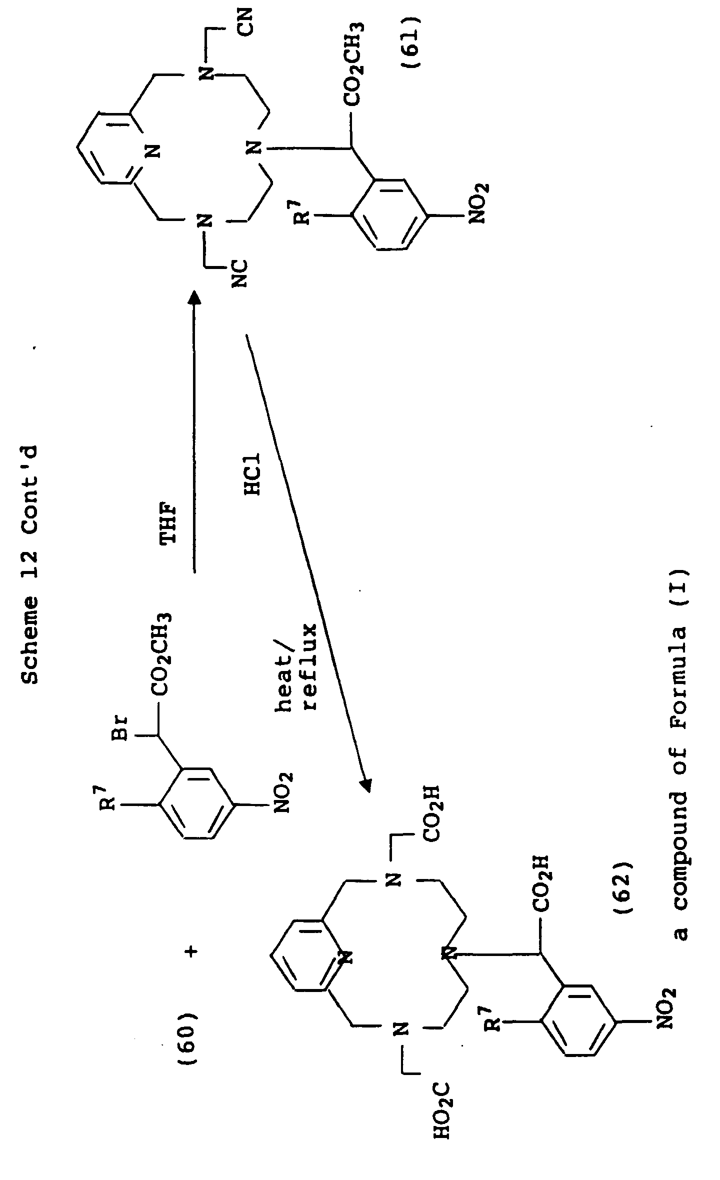

In the above Schemes, the general process description illustrates specific steps that may be used to accomplish a desired reaction step. The general description of these process steps follows.

The synthetic Scheme 1 begins with a halogenation of commercially available bis-pyridyl alcohol (1) using thionyl chloride. Similar procedures for converting an alcohol to an electrophilic substrate, such as treatment with toluenesulfonyl chloride, HBr or HCl, should also result in a similarly reactive product which would work well in subsequent ring closure reactions. Macrocyclization procedures are numerous in the literature and the desired tetraazamacrocycle (3) was prepared according tothe method of Stetter et al., *Tetrahedron* 37, 767-772(1981). More general procedures have since been published which give good yields of similar macrocycles using milder conditions [A. D. Sherry et al., *J. Org. Chem*. 54, 2990-2992 (1989)]. Detosylation of the intermediate macrocycle [(3) to yield (4)] was accomplished under acidic conditions in good yield. Reductive detosylation procedures are also well known in the literature and can be adapted to the present reaction sequence.

Schemes 10, 11 and 12 delineate a synthetic approach which introduces an aromatic nitrobenzyl substituent at one of the macrocyclic nitrogen positions. Typically, the macrocyclic amine is mono-N-functionalized in an organic solvent such as acetonitrile or DMF at room temperature using a non-nucleophilic base such as potassium carbonate. Additional functionalization of the remaining nitrogen positions is then preformed by methods and conditions described in previous Schemes. After the introduction of the desired chelating moieties, the nitro group is reduced using platinum oxide and hydrogen in water. In this form, the chelating agent is compatible with conjugation techniques which will enable attachment to larger synthetic or natural molecules.

The metal ions used to form the complexes of this invention are Gd⁺³, Mn⁺², Fe⁺³ and available commercially, e.g. from Aldrich Chemical Company. The anion present is halide, preferably chloride, or salt free (metal oxide).

A "paramagnetic nuclide" of this invention means a metal ion which displays spin angular momentum and/or orbital angular momentum. The two types of momentum combine to give the observed paramagnetic moment in a manner that depends largely on the atoms bearing the unpaired electron and, to a lesser extent, upon the environment of such atoms. The paramagnetic nuclides found to be useful in the practice of the invention are gadolinium (Gd⁺³), iron (Fe⁺³) and manganese (Mn⁺²) with Gd⁺³ being preferred.

The complexes are prepared by methods well known in the art. Thus, for example, see Chelating Agents and Metal Chelates, Dwyer & Mellor, Academic Press (1964), Chapter 7. See also methods for making amino acids in Synthetic Production and Utilization of Amino Acids, (edited by Kameko, et al.) John Wiley & Sons (1974). An example of the preparation of a complex involves reacting a bicyclopolyazamacrocyclophosphonic acid with the metal ion under aqueous conditions at a pH from 5 to 7. The complex formed is by a chemical bond and results in a stable paramagnetic nuclide composition, e.g. stable to the disassociation of the paramagnetic nuclide from the ligand.

The complexes of the present invention are administered at a ligand to metal molar ratio of at least about 1:1, preferably from 1:1 to 3:1, more preferably from 1:1 to 1.5:1. A large excess of ligand is undesirable since uncomplexed ligand may be toxic to the animal or may result in cardiac arrest or hypocalcemic convulsions.

The antibodies or antibody fragments which may be used in the conjugates described herein can be prepared by techniques well known in the art. Highly specific monoclonal antibodies can be produced by hybridization techniques well known in the art, see for example, Kohler and Milstein [*Nature*, 256, 495-497 (1975); and *Eur. J. Immunol*., 6, 511-519 (1976)]. Such antibodies normally have a highly specific reactivity. In the antibody targeted conjugates, antibodies directed against any desired antigen or hapten may be used. Preferably the antibodies which are used in the conjugates are monoclonal antibodies, or fragments thereof having high specificity for a desired epitope(s). Antibodies used in the present invention may be directed against for example, tumors, bacteria, fungi, viruses, parasites, mycoplasma, differentiation and other cell membrane antigens, pathogen surface antigens, toxins, enzymes, allergens, drugs and any biologically active molecules. Some examples of antibodies or antibody fragments are 1116-NS-19-9, 1116-NS-3d, 703D4, 704A1, CC49, CC83 and 872.3. All of these antibodies have been deposited in ATCC. A more complete list of antigens can be found in U.S. Patent 4,193,983. The conjugates of the present invention are particularly preferred for the diagnosis of various cancers.

This invention is used with a physiologically acceptable carrier, excipient or vehicle therefor. The methods for preparing such formulations are well known. The formulations may be in the form of a suspension, injectable solution or other suitable formulations. Physiologically acceptable suspending media, with or without adjuvants, may be used.

An "effective amount" of the formulation is used for diagnosis. The dose will vary depending on the disease and physical parameters of the animal, such as weight. *In vivo* diagnostics are also contemplated using formulations of this invention.

Other uses of some of the chelants of the present invention may include the removal of undesirable metals (i.e. iron) from the body, attachment to polymeric supports for various purposes, e.g. as diagnostic agents, and removal of metal ion by selective extraction. The ligands of Formula (I) having in at least two R terms T equal to P(O)R¹OH may be used for metal ion control as scale inhibitors. It is likely that these ligands could be used in less than stoichiometric amounts. Similar uses are known for compounds described in U.S. Patents 2,609,390; 3,331,773; 3,336,221; and 3,434,969.

## Claims

1. A complex which comprises a bicyclopolyazamacrocyclocarboxylic acid compound of the formula 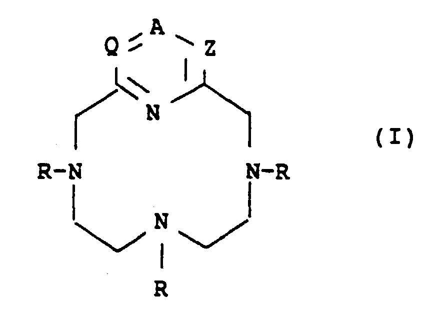 wherein:
R is 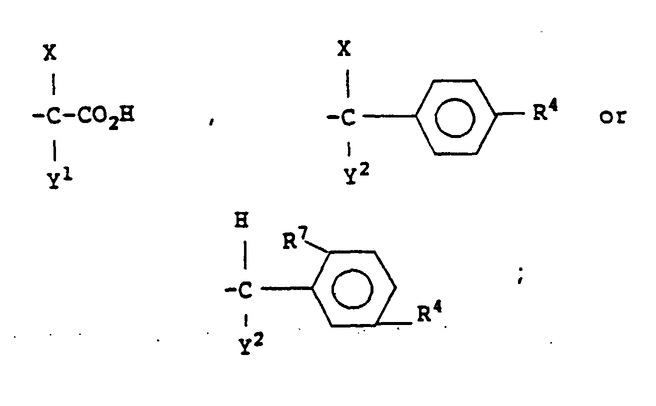 where:
X and Y¹ are independently H, OH or C₁-C₃ alkyl;
Y² is H or COOH;
R⁷ is H, OH or OCH₃; and
R⁴ is H, NO₂, NH₂, isothiocyanato, semicarbazido, thiosemicarbazido, maleimido, bromoacetamido or carboxyl;
with the proviso that at least two R terms must be 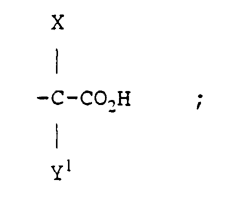
A = CR, N, C-Br, C-Cl, C-OR¹, C-OR², N⁺-R³X⁻, or 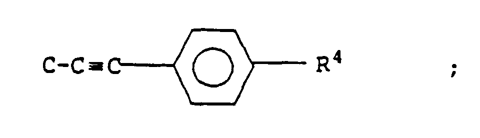
where: R¹ is H, C₁-C₅ alkyl, benzyl, or benzyl substituted with at least one R⁴;
R² is C₁-C₁₆ alkylamino;
R³ is C₁-C₁₆ alkyl, benzyl, or benzyl substituted with at least one R⁴;
X⁻ is Cl⁻, Br, I⁻ or H₃CCO₂⁻;
Q and Z independently are CH, N, N⁺-R³X⁻, C-CH₂-OR¹ or C-C(O)-R⁵;
R⁵ is -O-(C₁-C₃ alkyl), OH or NHR⁶;
R⁶ is C₁-C₅ alkyl or a biologically active material; with the proviso that:
a) when Q, A or Z is N or N⁺-R³X⁻, then the other two groups must be CH;
b) when A is C-Br, C-Cl, C-OR¹ or C-OR², then both Q and Z must be CH;
c) the sum of the R², R⁴ and R⁶ terms may not exceed one, and one of the R², R⁴, and R⁶ terms must be present, and;
d) only one of Q or Z can be C-C(O)-R⁵ and when one of Q or Z is C-C(O)-R⁵, then A must be CH; and
complexed with a metal ion selected from Gd⁺³, Mn⁺² or Fe⁺³; or
pharmaceutically-acceptable salts thereof.

2. A complex of Claim 1 wherein the metal is Gd⁺³.

3. A complex of Claim 1 wherein A, Q and Z are CH; and X, Y¹ and Y² are H.

4. A complex of Claim 1 wherein X, Y¹ and Y² are H.

5. A complex of Claim 1 wherein A, Q and Z are CH.

6. A complex of Claim 1 wherein A, Q and Z are CH; and one R term is 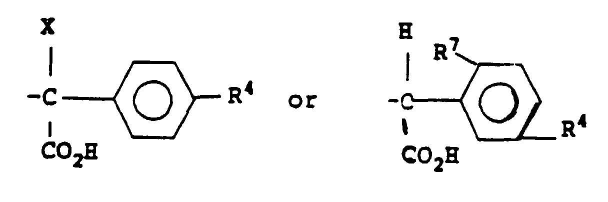 where: X, R⁴ and R⁷ are defined as in Claim 1.

7. A complex of Claim 1 wherein A is C-OR¹, C-OR², where R¹ and R² are defined as in Claim 1 or 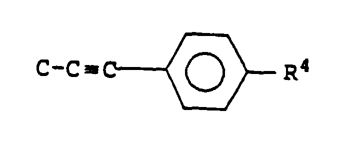 where R⁴ is defined as in Claim 1.

8. A complex of Claim 1 wherein A is CH, and one of Q or Z is CH and the other is C-C(O)R⁵ or C-CH₂-OR¹, where R¹ and R⁵ are as defined in Claim 1.

9. A complex of Claim 8 wherein R⁵ is NHR⁶, where R⁶ is a biologically active material.

10. A conjugate comprising a bicyclopolyazamacrocyclocarboxylic acid complex as claimed in Claim 1, covalently attached to a biologically active material.

11. A conjugate of Claim 10 wherein the biologically active material is a dextran, a peptide, a molecule that has specific affinity for a receptor, or an antibody or antibody fragment.

12. A conjugate of Claim 11 wherein the antibody or antibody fragment is a monoclonal antibody or fragment thereof.

13. A conjugate of Claim 12 wherein the antibody or antibody fragment is B72.3.

14. A conjugate of any one of Claims 10-13 wherein the metal ion is Gd⁺³ and the complex is defined as in any one of Claims 2 to 9 excluding the compound of Claim 8 in which one of Q and Z is C-CH₂-OR¹.

15. A pharmaceutical formulation comprising a complex as claimed in any one of Claims 1-9 with a pharmaceutically-acceptable carrier.

16. A pharmaceutical formulation comprising a conjugate as claimed in any one of Claims 10-14 with a pharmaceutically-acceptable carrier.

17. The complex as claimed in any one of Claims 1-9 for use as a pharmaceutical.

18. The conjugate as claimed in any one of Claims 10-14 for use as a pharmaceutical.

19. A kit for use as a diagnostic agent having as an ingredient a ligand as claimed in any one of Claims 1 to 9.

20. A process for preparing a complex as claimed in Claim 1 which comprises reacting a bicyclopolyazamacrocyclo-carboxylic acid compound as claimed in Claim 1 with a metal ion selected from Gd⁺³, Mn⁺² or Fe⁺³ under aqueous conditions at a pH from 5 to 7.

## Patentansprüche

1. Komplex, welcher eine BicyclopolyazamacrocyclocarbonsäureVerbindung der Formel 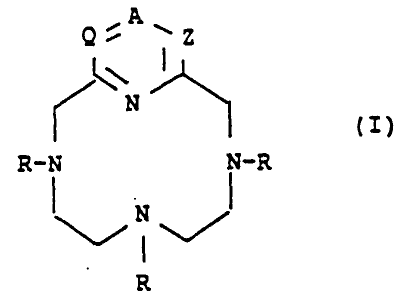 umfaßt, worin: R 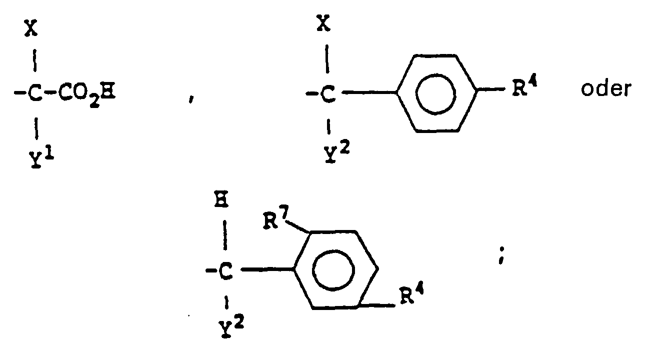 ist, worin:
X und Y¹ unabhängig H, OH oder C₁-C₃-Alkyl sind;
Y² H oder COOH ist;
R⁷ H, OH oder OCH₃ ist; und
R⁴ H, NO₂, NH₂, Isothiocyanat, Semicarbazid, Thiosemicarbazid, Maleimid, Bromacetamid oder Carboxyl ist;
mit der Maßgabe, daß mindestens zwei Terme R 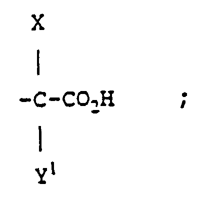 sein müssen;
A = CH, N, C-Br, C-Cl, C-OR¹, C-OR², N⁺-R³X⁻ oder 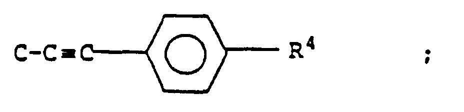
worin: R¹ H, C₁-C₅-Alkyl, Benzyl oder Benzyl substituiert mit mindestens einem R⁴ ist;
R² C₁-C₁₆-Alkylamino ist;
R³ C₁-C₁₆-Alkyl, Benzyl oder Benzyl substituiert mit mindestens einem
R⁴ ist;
X⁻ Cl⁻, Br⁻, I⁻ oder H₃CCO₂⁻ ist;
Q und Z unabhängig CH, N, N⁺-R³X; C-CH₂-OR¹ oder C-C(O)-R⁵ sind;
R⁵-O-(C₁-C₃-Alkyl), OH oder NHR⁶ ist;
R⁶ C₁-C₅-Alkyl oder ein biologisch aktives Material ist;
mit der Maßgabe, daß:
a) wenn Q, A oder Z N oder N⁺-R³X⁻ ist, dann müssen die anderen zwei Gruppen CH sein;
b) wenn A C-Br, C-Cl, C-OR¹ oder C-OR² ist, dann müssen sowohl Q als auch Z CH sein;
c) die Summe der Terme R², R⁴ und R⁶, eins nicht übersteigen darf und einer der Terme R², R⁴ und R⁶ vorliegen muß; und
d) nur eines von Q oder Z C-C(O)-R⁵ sein kann und wenn eines von Q oder Z C-C(O)-R⁵ ist, dann muß A CH sein; und
die mit einem Metallion komplexiert ist, ausgewählt aus Gd³⁺, Mn²⁺ oder Fe³⁺; oder
pharmazeutisch annehmbare Salze davon.

2. Komplex nach Anspruch 1, worin das Metall Gd³⁺ ist.

3. Komplex nach Anspruch 1, worin A, Q und Z OH sind und X, Y¹ und Y² H sind.

4. Komplex nach Anspruch 1, worin X, Y¹ und Y²H sind.

5. Komplex nach Anspruch 1, worin A, Q und Z CH sind.

6. Komplex nach Anspruch 1, worin A, Q und Z OH sind; und ein Term R 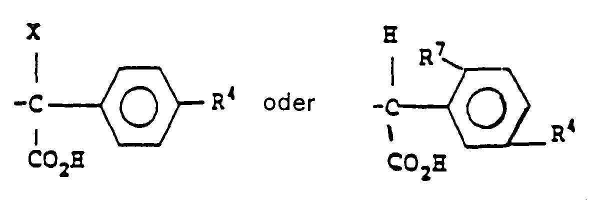 ist, worin: X, R⁴ und R⁷ wie in Anspruch 1 definiert sind.

7. Komplex nach Anspruch 1, worin A C-OR¹, C-OR² ist, worin R¹ und R² wie in Anspruch 1 definiert sind oder 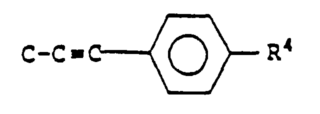 ist, worin R⁴ wie in Anspruch 1 definiert ist.

8. Komplex nach Anspruch 1, worin A CH ist und eines von Q oder Z CH ist und das andere C-C(O)R⁵ oder C-CH₂-OR¹ ist, worin R¹ und R⁵ wie in Anspruch 1 definiert sind.

9. Komplex nach Anspruch 8, worin R⁵ NHR⁶ ist, worin R⁶ ein biologisch aktives Material ist.

10. Konjugat umfassend einen
Bicyclopolyazamacrocyclocarbonsäurekomplex nach Anspruch 1, kovalent gebunden an ein biologisch aktives Material.

11. Konjugt nach Anspruch 10, worin das biologisch aktive Material ein Dextran, ein Peptid, ein Molekül, das eine spezifische Affinität für einen Rezeptor aufweist, oder ein Antikörper oder ein Antikörperfragment ist.

12. Konjugat nach Anspruch 11, worin der Antikörper oder das Antikörperfragment ein monoklonaler Antikörper oder ein Fragment davon ist.

13. Konjugat nach Anspruch 12, worin der Antikörper oder das Antikörperfragment B72.3 ist.

14. Konjugat nach einem der Ansprüche 10 bis 13, worin das Metallion Gd³⁺ ist und der Komplex wie in einem der Ansprüche 2 bis 9 definiert ist, ausgenommen die Verbindung von Anspruch 8, in der eines von Q und Z C-CH₂-OR¹ ist.

15. Pharmazeutische Formulierung umfassend einen Komplex nach einem der Ansprüche 1 bis 9 mit einem pharmazeutisch annehmbaren Träger.

16. Pharmazeutische Formulierung umfassend ein Konjugat nach einem der Ansprüche 10 bis 14 mit einem pharmazeutisch annehmbaren Träger.

17. Komplex nach einem der Ansprüche 1 bis 9 zur Verwendung als Pharmazeutikum.

18. Konjugat nach einem der Ansprüche 10 bis 14 zur Verwendung als Pharmazeutikum.

19. Kit zur Verwendung als diagnostisches Mittel, der als Bestandteil einen Liganden nach einem der Ansprüche 1 bis 9 enthält.

20. Verfahren zur Herstellung eines Komplexes nach Anspruch 1, welches das Umsetzen einer
Bicyclopolyazamacrocyclocarbonsäureverbindung nach Anspruch 1 mit einem Metallion, ausgewählt aus Gd³⁺, Mn²⁺ oder Fe³⁺ unter wäßrigen Bedingungen bei einem pH-Wert von 5 bis 7 umfaßt.

## Revendications

1. Complexe qui comporte un composé de type acide bicyclopolyazamacrocyclocarboxylique, de formule 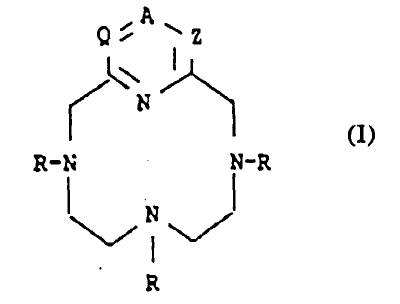 dans laquelle R représente 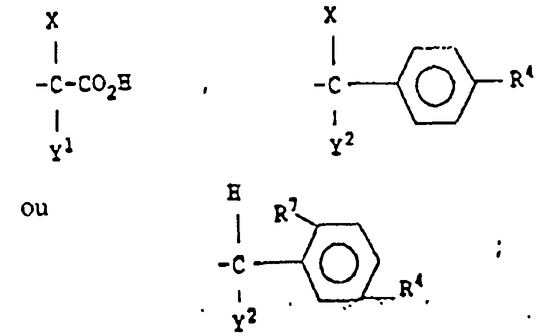 où X et Y¹ représentent chacun, indépendamment, un atome d'hydrogène ou un groupe hydroxyle ou alkyle en C₁₋₃,
Y² représente un atome d'hydrogène ou un groupe carboxyle,
R⁷ représente un atome d'hydrogène ou un groupe hydroxyle ou méthoxy,
et R⁴ représente un atome d'hydrogène ou un groupe nitro, amino, isothiocyanato, semicarbazido, thiosemicarbazido, maléimido, bromoacétamido ou carboxyle,
sous réserve qu'au moins deux des symboles R représentent obligatoirement 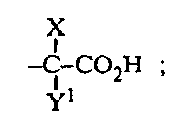
A représente CH, N, C-Br, C-Cl, C-OR¹, C-OR², N⁺-R³X⁻ ou 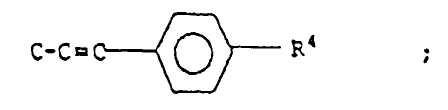
où R¹ représente un atome d'hydrogène ou un groupe alkyle
en C₁₋₅, benzyle, ou benzyle portant au moins un substituant R⁴*,*
R² représente un groupe (alkyle en C₁₋₁₆)amino,
R³ représente un groupe alkyle en C₁₋₁₆, benzyle, ou benzyle portant au moins un substituant R⁴,
et X⁻ représente un ion Cl⁻, Br⁻, I⁻ ou H₃CCOC₂⁻;
et Q et Z représentent chacun, indépendamment, CH, N, N⁺-R³X⁻; C-CH₂-OR¹ ou C-C(O)-R⁵,
où R⁵ représente un groupe hydroxyle ou alcoxy en C₁₋₃,
ou encore NHR⁶, où R⁶ représente un groupe alkyle en C₁₋₅
ou un reste de substance biologiquement active ;
avec les réserves suivantes :
a) si Q, A ou Z représente N ou N⁺-R³X⁻, les deux autres symboles représentent obligatoirement CH ;
b) si A représente C-Br, C-Cl, C-OR¹ ou C-OR², Q et Z représentent tous les deux obligatoirement CH ;
c) le nombre total de symboles R², R⁴ et R⁶ ne doit pas dépasser 1, mais il y a obligatoirement un symbole R², R⁴ ou R⁶ ; et
d) un seul des symboles Q et Z peut représenter C-C(O)- R⁵, et si l'un des symboles Q et Z représente C-C(O)-R⁵, A représente obligatoirement CH ;
ce composé étant complexé avec un ion métallique choisi parmi Gd³⁺, Mn²⁺ ou Fe³⁺;
et sels d'un tel composé, admissibles en pharmacie.

2. Complexe conforme à la revendication 1, dans lequel l'ion métallique est Gd³⁺.

3. Complexe conforme à la revendication 1, dans lequel A, Q et Z représentent CH, et X, Y¹ et Y² représentent des atomes d'hydrogène.

4. Complexe conforme à la revendication 1, dans lequel X, Y¹ et Y² représentent des atomes d'hydrogène.

5. Complexe conforme à la revendication 1, dans lequel A, Q et Z représentent CH.

6. Complexe conforme à la revendication 1, dans lequel A, Q et Z représentent CH, et l'un des symboles R représente 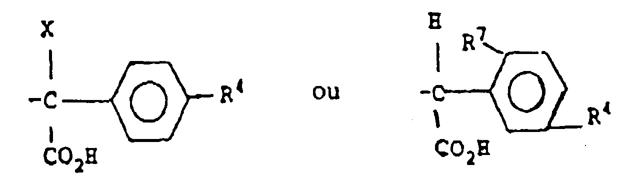 où X, R⁴ et R⁷ ont les significations indiquées dans la revendication 1.

7. Complexe conforme à la revendication 1, dans lequel A représente C-OR¹ ou C-OR², où R¹ et R² ont les significations indiquées dans la revendication 1, ou encore 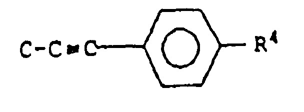 où R⁴ a la signification indiquée dans la revendication 1.

8. Complexe conforme à la revendication 1, dans lequel A représente CH, et l'un des symboles Q et Z représente CH et l'autre représente C-C(O)-R⁵ ou C-CH₂-OR¹, où R¹ et R⁵ ont les significations indiquées dans la revendication 1.

9. Complexe conforme à la revendication 8, dans lequel R⁵ représente NHR⁶, où R⁶ représente un reste de substance biologiquement active.

10. Conjugué comportant un complexe d'un acide bicyclopolyazamacrocyclocarboxylique, conforme à la revendication 1 et lié par liaison covalente à une substance biologiquement active.

11. Conjugué conforme à la revendication 10, dans lequel la substance biologiquement active est un dextrane, un peptide, une molécule présentant une affinité spécifique pour un récepteur, ou un anticorps ou fragment d'anticorps.

12. Conjugué conforme à la revendication 11, dans lequel l'anticorps ou fragment d'anticorps est un anticorps monoclonal ou un fragment d'un tel anticorps.

13. Conjugué conforme à la revendication 12, dans lequel l'anticorps ou fragment d'anticorps est B72.3.

14. Conjugué conforme à l'une des revendications 10 à 13, dans lequel l'ion métallique est Gd³⁺ et le complexe est conforme à l'une des revendications 2 à 9, à l'exclusion des composés conformes à la revendication 8 chez lesquels l'un des symboles Q et Z représente C-CH₂-OR¹.

15. Formulation pharmaceutique qui comprend un complexe conforme à l'une des revendications 1 à 9, joint à un véhicule admissible en pharmacie.

16. Formulation pharmaceutique qui comprend un conjugué conforme à l'une des revendications 10 à 14, joint à un véhicule admissible en pharmacie.

17. Complexe conforme à l'une des revendications 1 à 9, destiné à être employé comme agent pharmaceutique.

18. Conjugué conforme à l'une des revendications 10 à 14, destiné à être employé comme agent pharmaceutique.

19. Trousse destinée à être utilisée comme outil de diagnostic, dont l'un des ingrédients est un ligand défini selon l'une des revendications 1 à 9.

20. Procédé de préparation d'un complexe conforme à la revendication 1, qui comporte le fait de faire réagir un composé de type acide bicyclopolyazamacrocyclocarboxylique, défini dans la revendication 1, avec un ion métallique choisi parmi Gd³⁺, Mn²⁺ ou Fe³⁺, dans un milieu aqueux dont le pH vaut de 5 à 7.
